# EUROPÄISCHE PATENTSCHRIFT

(11) **EP 0 684 023 B1**
(45) Veröffentlichungstag und Bekanntmachung des Hinweises auf die Patenterteilung: **05.04.2000**
(21) Anmeldenummer: 95101976.9
(22) Anmeldetag: 14.02.1995
(51) Int. Cl.: A61F 2/36

(54) **Femurprothese**
Femoral prosthesis
Prothèse fémorale

(30) Priorität: 26.05.1994 CH 162994
(43) Veröffentlichungstag der Anmeldung: 29.11.1995
(73) Patentinhaber: Hermann, Werner, CH-6312 Steinhausen (CH)
(72) Erfinder: Hermann, Werner, CH-6312 Steinhausen (CH)
(74) Vertreter: OK pat AG

(56) Entgegenhaltungen:
- EP-A- 0 244 610
- EP-A- 0 366 945
- WO-A-89/08436
- FR-A- 2 295 730
- GB-A- 2 028 137
- US-A- 5 133 766

## Beschreibung

Die Erfindung betrifft eine Femurprothese nach dem Oberbegriff des Anspruchs 1.

Femurprothesen dieser Art sind beispielsweise aus der EP-A-0 244 610 oder EP-A-0 366 945 bekannt. Während es sich im ersten Fall speziell um das Einsetzen der Prothese in den Knochen und allenfalls das nachträgliche Entfernen daraus handelt, wird gemäss der zweitgenannten Veröffentlichung besonders ein gutes Einpassen des Prothesenschaftes in die vorbereitete Implantationshöhlung des Knochens angestrebt. Letzteres soll mittels einer Mehrzahl von separaten Rippenteilen erreicht werden, die in Längsnuten an den Seiten des Schaftes eingesetzt und geführt sind. Die beschriebene, mehrteilige Prothesenkonstruktion ist allerdings sehr kompliziert und wegen der erforderlichen Präzision der Nut-Feder-Passungen teuer in der Herstellung. Abgesehen von weiteren Nachteilen, die die Arbeit des Chirurgen betreffen, erscheint auch der sichere Sitz des Prothesenschaftes im Knochen zweifelhaft, weil die an der Prothese angreifenden Kräfte und Momente praktisch ausschliesslich über die seitlich abstehenden Rippen auf den Knochen übertragen werden.

Aus der GB-A-2 028 137 und aus der FR-A-2 295 730 sind weitere Femurprothesen bekannt, mit sich zum distalen Ende verjüngenden Schäften und mit gestuften Bereichen an den Schmalseiten. Die Schmalseiten nehmen aber im Bereich der Stufen nicht durchwegs eine Stellung ein, in der sie mit der Symmetrieebene des Implantatträgers einen konstanten oder mindestens nahezu konstanten Winkel einschliessen. Neben dem Nachteil der sehr aufwendigen Fertigung hat diese Formgebung den weiteren Nachteil, dass die beim Tragen der Femurprothese die bei den hauptsächlich auftretenden Drehbelastungen auftretenden Kräfte eine Lockerung der Fixierung des Schaftes zur Folge haben können.

Es ist daher Aufgabe der Erfindung, ausgehend von einem Stand der Technik gemäss der GB-A-2 028 137, eine Femurprothese zu schaffen, bei welcher die eben erwähnten Nachteile vermieden werden.

Diese Aufgabe wird erfindungsgemäss durch die Merkmale des kennzeichnenden Teils des Anspruchs **1** gelöst. Vorteilhafte Weiterbildungen der erfindungsgemässen Femurprothese sind durch die Ansprüche **2** bis **5** definiert.

Mit der vorliegenden Erfindung wird eine solche Gestaltung der Femurprothese erreicht, dass ein vollflächiger, satter und dauerhafter Sitz des Prothesenschaftes gewährleistet ist, so dass sowohl Belastungen in Schaft-Längsrichtung wie auch besonders die erheblichen und wechselnden, von der Gelenkkugel her auftretenden Drehmomente sicher auf den Oberschenkelknochen übertragen werden. Auch ist dabei die erforderliche Implantationshöhlung nicht grösser als unmittelbar durch den Schaftquerschnitt bedingt.

Die mit der Erfindung erzielten Vorteile werden insbesondere darin gesehen, dass die erwähnten Stufen einerseits eine mit dem Knochen eng "verzahnte" Auflage bilden, und zwar praktisch im Bereich der Wirkungslinie der an der Gelenkkugel angreifenden, generell vertikal gerichteten Kräfte. Damit wird eine besonders wirksame Abstützung auf dem Femur erreicht, d.h. ein weiteres Eintreiben bzw. Einsinken des Schaftes in den Knochen unter Last sicher verhindert. Anderseits werden durch die angegebene Neigung der Stufen auch die an der Prothese angreifenden Drehmomente wirksam auf das Femur übertragen (zusätzlich zu der durch den Vierkant-Querschnitt des Schaftes gegebenen Drehverbindung); infolge der natürlichen Neigung zwischen Oberschenkel und Rumpf ist nämlich an dem schräg nach oben und medial bezüglich der Schaft-Achse abstehenden Hals der Prothese - gleich wie am natürlichen Oberschenkelhals - bei Belastung des Gelenkes immer eine Kraftkomponente wirksam, die von vorn nach hinten gerichtet ist. Die Schieflage der Stufen ist nun so, dass sie "Anstellflächen" bilden, welche (bei gleichzeitig vorhandener Vertikalbelastung) Gleitbewegungen entlang den Stufenflächen entgegenwirken. Wesentlich ist auch, dass der gestufte Bereich bzw. die dort stattfindenden Kraft- und Momentübertragungen sich weit oben in der Nähe des proximalen Endes des Femurs und damit in Bereichen fester und reichlich vorhandener Knochensubstanz befinden.

Bei einer bevorzugten Ausführungsform der erfindungsgemässen Femurprothese ist auch an der lateralen Schmalseite eine entsprechende Stufung vorgesehen, und zwar an einer Anschrägung im oberen Bereich der Schmalseite, die oben im bearbeiteten Medullarkanal des Femurs einen Raum freilässt.

Nachstehend wird die Erfindung anhand eines Ausführungsbeispiels in Verbindung mit der Zeichnung näher erläutert.
- Fig. 1: ist die Ansicht von hinten,
- Fig. 2: die Ansicht auf die laterale Schmalseite, und
- Fig. 3: die Draufsicht auf eine für das rechte Femur bestimmte erfindungsgemässe Femurprothese.

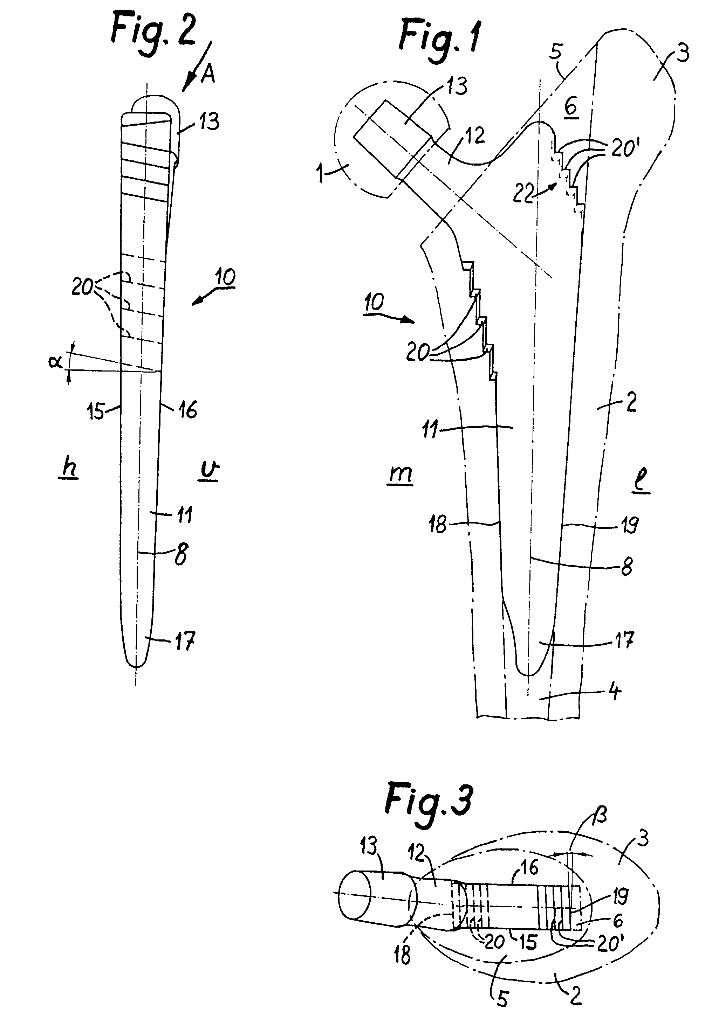

Zwecks leichterer Orientierung sind in Fig. 1 die Lateralseite mit l und die Medialseite mit m, und in Fig. 2 die Vorderseite mit v sowie die Hinterseite mit h bezeichnet. Alle Aussagen über die hier dargestellte rechte Femurprothese gelten natürlich sinngemäss für eine linke Femurprothese, die bezüglich der Medianebene des Körpers symmetrich zur rechten Femurprothese gestaltet ist.

In den Fig. 1 und 3 ist der obere Teil des Femurs 2, in das die Prothese eingesetzt ist, schematisch und strichpunktiert angedeutet. Zum Einsetzen des Prothesenschaftes wird vom Bereich des Trochanter major 3 her der Medullarkanal 4 des Knochens vom Chirurgen passend erweitert, und im allgemeinen wird eine zur Längsrichtung des Femurs 2 schief stehende Resektionsebene 5 gebildet.

Die Femurprothese 10 besteht aus dem vierkantigen Schaft 11 mit etwa rechteckigem Querschnitt, und dem zur Schaft-Längsachse 8 abgewinkelten Hals 12 (Collum-Corpus-Winkel), dessen Ende einen Konus 13 zur Aufnahme der Gelenkkugel 1 aufweist. Wie aus den Fig. 1 und 3 ersichtlich, ist der Hals 12 gegenüber den Flachseiten 15, 16 des Schaftes 11 leicht nach vorn abgeknickt (Torsionswinkel). In Fig. 2 ist eine Hauptrichtung der an der Gelenkkugel 1 vom Becken her wirkenden Belastung mit dem Pfeil A eingezeichnet. Diese Richtung ist im wesentlichen durch die Neigung zwischen Rumpf und Oberschenkel gegeben und weist praktisch immer eine vertikale Komponente und eine von vorn nach hinten gerichtete horizontale Komponente auf.

Der vierkantige, blattförmige Schaft 11 (auch "Schaftblatt" genannt) ist, wie dargestellt, gegen das distale Ende 17 hin verjüngt, indem die Schmalseiten 18, 19 und vorzugsweise auch die Flachseiten 15, 16 von oben nach unten zusammenlaufen. Die mediale Schmalseite 18 ist in einem oberen Bereich - vor dem Uebergang zum Hals 12 und noch innerhalb des Femurs 2 - gestuft. Die kantigen Stufen 20 sind dabei zur Längsachse 8 des Schaftes so geneigt, dass sie von hinten (Flachseite 15) nach vorn (Flachseite 16) abfallen; der Neigungswinkel α der Stufen 20 (Fig. 1) beträgt dabei vorzugsweise etwa 5 bis 20°. Dank solchen Stufen 20 werden die an der Gelenkkugel 1 angreifenden vertikalen Kräfte praktisch in ihrer Wirkungsrichtung in das Femur 2 eingeleitet, und zwar in einem Bereich mit im allgemeinen reichlicher und tragfähiger Knochensubstanz. Ebenso werden den Vertikalkräften überlagerte Horizontalkomponenten bzw. Torsionsmomente infolge der Neigung der Stufen 20 bevorzugt in deren Bereich auf den Knochen übertragen, also bereits oben im Femur, und entsprechend weniger entlang den Schaftseiten mit entsprechender Torsionsbeanspruchung über die ganze Länge des Schaftes 11.

Wie vor allem aus der Fig. 1 hervorgeht, ist im oberen Bereich der lateralen Schmalseite 19 vorzugsweise eine Anschrägung 22 vorgesehen, die in analoger Weise wie die mediale Schmalseite 18 mit geneigten Stufen 20' versehen ist. Die Anschrägung 22 lässt oben im bearbeiteten, erweiterten Medullarkanal 4 einen Raum 6 frei. Dieser Raum 6 kann bei der Operation teilweise mit natürlicher Knochensubstanz angefüllt werden; bei günstigem Heilungsverlauf kann damit gerechnet werden, dass diese mit dem umgebenden und neu gebildetem Knochen verwächst, wobei dann die Stufen 20' ebenfalls entsprechend zum Tragen kommen.

Ein weiteres zweckmässiges Gestaltungsmerkmal, das den Querschnitt des Schaftblattes 11 betrifft, ist aus Fig. 3 ersichtlich: Wie mit dem Winkel β angedeutet, ist der Schaftquerschnitt nicht streng rechteckig, sondern durch ein schiefwinkliges Parallelogramm (ein Rhomboid oder ggf. einen Rhombus) bestimmt. Dabei befinden sich die beiden spitzen Winkel des Parallelogramms jeweils medial vorn und lateral hinten. Der Winkel β, um welchen die beiden spitzen Winkel von 90° abweichen, beträgt vorzugsweise etwa 2 bis 10°. Durch diese Gestaltung bilden die beiden Schmalseiten 18 und 19 ebenfalls "Anstellflächen", die zur Drehmoment-Uebertragung auf das Femur weiter beitragen.

## Patentansprüche

1. Femurprothese
- mit einem zum Einsetzen in den Oberschenkelknochen bestimmten vierkantigen Schaft (**11**), der zum distalen Ende hin verjüngt ist und eine hintere Flachseite (**15**), eine vordere Flachseite (**16**), eine mediale Schmalseite (**18**) und eine laterale Schmalseite (**19**) aufweist,
- mit einem vom Schaft (**11**) medial abstehenden, zur Aufnahme einer Gelenkkugel bestimmten Hals (**12**),
- wobei die mediale Schmalseite (**18**) im oberen Bereich Stufen (**20**) aufweist, von welchen jede zur Längsachse (**8**) des Schaftes (**11**) von hinten nach vorne abfallend geneigt ist,
**dadurch gekennzeichnet,**
- dass die Stufen (20) mediale vertikale Stufenflächen aufweisen, die mit der Hinterseite (h) einen Stumpfen Winkel bilden.

2. Femurprothese nach Anspruch 1, dadurch gekennzeichnet, dass eine Anschrägung (22) im oberen Bereich der lateralen Schmalseite (19), die oben im bearbeiteten Medullarkanal (4) des Femurs (2) einen Raum (6) freilässt, in entsprechender Weise wie der obere Bereich der medialen Schmalseite (18) gestuft ist.

3. Femurprothese nach Anspruch 1 oder 2, dadurch gekennzeichnet, dass der Neigungswinkel (α) der Stufen etwa 5 bis 15° beträgt.

4. Femurprothese nach Anspruch 1, dadurch gekennzeichnet, dass der Querschnitt des vierkantigen Schaftes (11) ein schiefwinkliges Parallelogramm (Rhombus oder Rhomboid) ist, dessen spitze Winkel sich medial vorn und lateral hinten befinden.

5. Femurprothese nach Anspruch 4, dadurch gekennzeichnet, dass die spitzen Winkel um einen Winkel (β) von etwa 2 bis 10° von 90° abweichen.

## Claims

1. Femur prosthesis
- having a four-edged shaft (11) which is intended to be inserted into the femur and tapers towards the distal end, and which has a posterior flat face (15), an anterior flat face (16), a medial narrow face (18) and a lateral narrow face (19),
- having a neck (12) which juts medially from the shaft (11) and is intended to receive the ball of a joint,
- the upper region of the medial narrow face (18) incorporating steps (20), each of which is sloped in a manner that falls away from back to front relative to the the longitudinal axis (8) of the shaft (11),
characterised in that
- the steps (20) have medial vertical step surfaces which form an obtuse angle with the posterior face (h).

2. Femur prosthesis according to claim 1, characterised in that an upwardly angled area (22) in the upper region of the lateral narrow face (19), which leaves free an area (6) at the top in the medullary canal (4) being worked on of the femur (2), is stepped in corresponding manner to the upper region of the medial narrow face (18).

3. Femur prosthesis according to claim 1 or 2, characterised in that the angle of inclination (α) of the steps is around 5 to 15°.

4. Femur prosthesis according to claim 1, characterised in that the cross-section of the four-edged shaft (11) is an oblique-angled parallelogram (rhombus or rhomboid) whose acute angles are situated medially at the front and laterally at the rear.

5. Femur prosthesis according to claim 4, characterised in that the acute angles deviate from 90° by an angle (β) of around 2 to 10°.

## Revendications

1. Prothèse fémorale
- avec un corps quadrangulaire (11) destiné à être inséré dans l'os du fémur, corps qui est rétréci vers l'extrémité distale et qui présente un côté plat arrière (15), un côté plat avant (16), un côté étroit médial (18) et un côté étroit latéral (19),
- avec un col (12), partant du corps (11) en direction médiale et destiné à recevoir une rotule d'articulation,
- le côté étroit médial (18) présentant dans la région supérieure des gradins (20) qui sont chacun inclinés vers l'axe longitudinal (8) du corps (11) avec une pente descendante de l'arrière vers l'avant,
caractérisée en ce que les gradins (20) présentent des faces médiales verticales de gradins, qui forment un angle obtus avec le côté arrière (h).

2. Prothèse fémorale selon la revendication 1, **caractérisée** en ce qu'un chanfreinage (22) dans la région supérieure du côté étroit latéral (19), qui laisse ouvert vers le haut un espace (6) dans le canal médullaire usiné (4) du fémur (2), est échelonné d'une manière correspondante à la région supérieure du côté étroit médial (18).

3. Prothèse fémorale selon la revendication 1 ou 2, **caractérisée** en ce que l'angle d'inclinaison (α) des gradins est compris entre 5 et 15°.

4. Prothèse fémorale selon la revendication 1, **caractérisée** en ce que la section transversale du corps quadrangulaire (11) est un parallélogramme à angle oblique (losange ou rhomboïde), dont les angles aigus se trouvent à l'avant en direction médiale et à l'arrière en direction latérale.

5. Prothèse fémorale selon la revendication 4, **caractérisée** en ce que les angles aigus diffèrent de 90° d'un angle (β) compris entre 2 et 10°.
